# FASCICULE DE BREVET EUROPEEN

(11) **EP 1 911 416 B1**
(45) Date de publication et mention de la délivrance du brevet: **18.08.2010**
(21) Numéro de dépôt: 07022675.8
(22) Date de dépôt: 10.06.2005
(51) Int. Cl.: A61F 2/00, A61B 17/00

(54) **Implant prothétique de soutènement sous-urétral**
Prothesenimplantat zur suburethralen Stützung
Prosthetic implant for sub-urethral support

(30) Priorité: 10.06.2004 FR 0406300
(43) Date de publication de la demande: 16.04.2008
(62) Demande divisionnaire de: 05777329.3
(73) Titulaire: Compagnie de Recherche en Composants, Implants et Materiels pour L'Application Clinique, 38200 Vienne (FR)
(72) Inventeur: Cristalli, Bernard, Gilbert, Robert, 91450 Soisy sur Seine (FR)
(74) Mandataire: Croonenbroek, Thomas Jakob

(56) Documents cités:
- WO-A-02/069781
- WO-A-03/092546
- US-A- 5 922 026
- US-A1- 2003 065 402

## Description

### DOMAINE TECHNIQUE

La présente invention se rapporte au domaine technique général des dispositifs implantables destinés à corriger l'incontinence urinaire, et notamment adaptés pour corriger l'incontinence urinaire d'effort chez la femme.

La présente invention concerne plus particulièrement un implant prothétique de soutènement sous-urétral selon la revendication 1.

### TECHNIQUE ANTERIEURE

L'incontinence urinaire correspond à une perte involontaire d'urine à travers l'urètre. Cette perte involontaire provient généralement du fait que les tissus responsables de la retenue des urines ne sont plus en mesure d'exercer des forces suffisantes pour s'opposer aux forces d'expulsion des urines. L'incontinence urinaire d'effort correspond en particulier à une perte d'urine non précédée de sensation de besoin, et qui ne se produit pas au repos mais pendant l'effort.

Parmi les traitements chirurgicaux proposés pour corriger l'incontinence urinaire, on distingue le traitement par soutènement urétral à l'aide d'une bandelette.

Il s'agit en effet d'une bandelette mise en place sans tension, sous la partie moyenne de l'urètre, et destinée à supporter l'urètre pendant l'effort, empêchant ainsi toute fuite d'urine. On distingue plusieurs types de bandelettes associées à des voies chirurgicales différentes. On connaît ainsi la bandelette TVT (Tension-free Vaginal Tape), associée au traitement chirurgical par la voie rétro-pubienne et la bandelette TOT (Trans-Obturator Tape) associée au traitement chirurgical par voie trans-obturatrice.

Dans le cas du traitement chirurgical utilisant la bandelette TVT, une incision est pratiquée dans le vagin, sous l'urètre, et deux incisions sont pratiquées dans la peau de l'abdomen. La bandelette TVT est placée en dessous de l'urètre à l'aide d'un instrument spécifique puis remonte de part et d'autre de la vessie pour venir s'ancrer dans la paroi abdominale.

Bien qu'efficace, la méthode utilisant la bandelette TVT est délicate à mettre en oeuvre et présente des risques opératoires non négligeables. En effet, il existe un risque de perforation de la vessie lors de la manipulation des aiguilles utilisées pour la pose de la bandelette. Des conséquences plus graves ont également été constatées tel qu'une perforation de l'artère iliaque ou encore de l'intestin grêle, entraînant des complications viscérales parfois mortelles. C'est la raison pour laquelle il est indispensable, avec cette méthode chirurgicale, de pratiquer une cystoscopie.

Dans le cas du traitement chirurgical utilisant la bandelette TOT, des incisions sont réalisées d'une part sur la paroi vaginale et d'autre part sur la face interne de chaque cuisse en regard des trous obturateurs. La bandelette TOT utilisée dans le cadre de cette méthode comporte une partie centrale disposée sous l'urètre et est conçue pour traverser les trous obturateurs et venir ensuite s'ancrer par ses extrémités au niveau de l'aine. Bien que cette méthode présente moins de risques de perforation d'organes nobles que la méthode par voie rétro-pubienne, elle n'élimine cependant pas complètement le risque de complications vasculaires en raison de la longueur du trajet occupé par la bandelette au sein du corps.

Le document WO-02/069781 décrit un implant conforme au préambule de la revendication 1.

### EXPOSE DE L'INVENTION

Les objets assignés à l'invention visent en conséquence à proposer un nouvel implant prothétique de soutènement sous-urétral ne comportant pas les inconvénients mentionnés précédemment et qui permette de corriger de manière efficace les incontinences urinaires d'effort en diminuant les risques de complication opératoire.

Un autre objet de l'invention vise à proposer un nouvel implant prothétique permettant de conduire une intervention simple, à risque minimum, et par voie vaginale exclusive.

Un autre objet de l'invention vise à proposer un nouvel implant prothétique susceptible d'être posé et maintenu sensiblement immobile sans tension.

Un autre objet de l'invention vise à proposer un nouvel implant prothétique qui soit particulièrement facile à mettre en place.

Un autre objet de l'invention vise à proposer un nouvel implant prothétique dont la fabrication est simple et reproductible.

### DESCRIPTIF SOMMAIRE DES DESSINS

D'autres avantages et objets de l'invention apparaîtront plus en détails à la lecture de la description qui suit, ainsi qu'à l'aide des dessins annexés fournis à titre purement illustratif et non limitatif, dans lesquels :
- La figure 1 illustre, selon une vue en coupe frontale, un implant prothétique de soutènement sous-urétral conforme à l'invention, dans sa position fonctionnelle au sein du corps de la patiente.
- La figure 2 illustre, selon une vue de dessus, un mode de réalisation d'un implant prothétique conforme à l'invention.
- La figure 3 illustre, selon une vue de face, l'implant prothétique représenté sur la figure 2.
- La figure 4 illustre, selon une vue de dessus, l'implant prothétique illustré sur la figure 2 dans un état précédant sa mise en forme terminale,
- La figure 5 illustre, selon une vue de face, un mode de réalisation d'un instrument chirurgical pour l'implantation d'un implant prothétique conforme à l'invention.
- La figure 6 illustre, selon une vue de dessus, l'instrument chirurgical représenté sur la figure 5.
- La figure 7 illustre, selon une vue en coupe suivant la ligne A-A illustrée sur la figure 6, un détail de réalisation de l'instrument chirurgical illustré sur la figure 6.
- La figure 8 illustre, selon une vue de dessus, une variante de réalisation de l'implant prothétique conforme à l'invention.
- La figure 9 illustre, selon une vue de dessus, une autre variante de réalisation de l'implant prothétique conforme à l'invention.
- La figure 10 illustre, selon une vue de face, l'implant prothétique représenté sur la figure 9.

### MEILLEURE MANIERE DE REALISER L'INVENTION

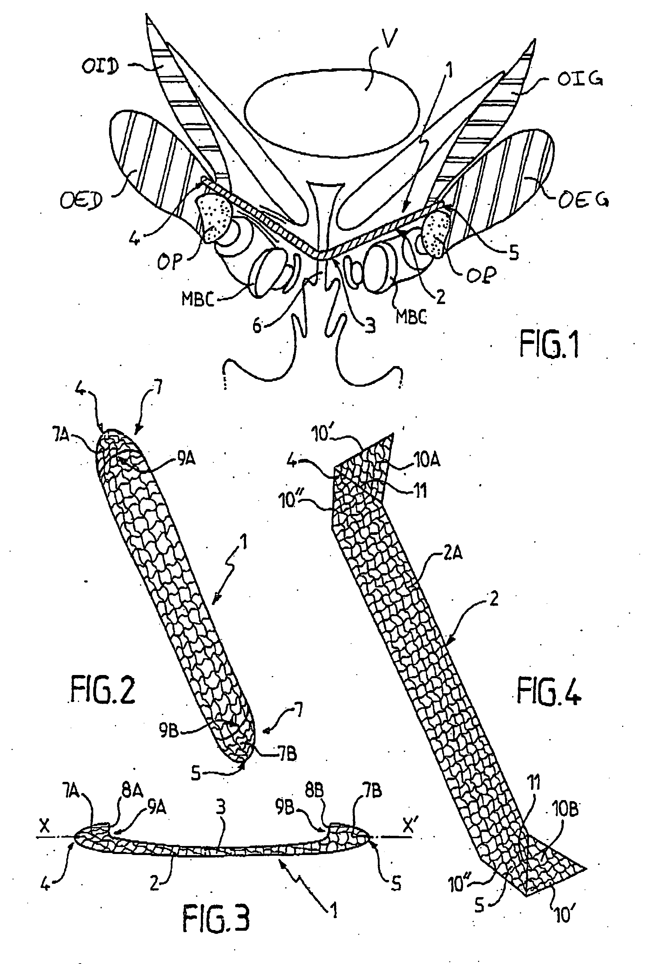

La figure 1 montre un implant prothétique conforme à l'invention destiné à être implanté dans le corps d'une patiente en vue de réaliser un soutènement de l'urètre permettant de corriger l'incontinence urinaire, et notamment l'incontinence urinaire d'effort chez la femme.

Le terme « *implant prothétique* » désigne ici une prothèse préfabriquée, destinée à être introduite à l'intérieur du corps de la patiente. La figure 1 illustre l'implant prothétique 1 dans sa position fonctionnelle, c'est-à-dire la position dans laquelle il va permettre de renforcer l'urètre et d'empêcher les fuites involontaires d'urine.

Selon l'invention, l'implant prothétique 1 comprend un élément allongé 2, se présentant par exemple sous la forme d'une bandelette textile, telle que celle illustrée sur la figure 2. Cette bandelette est de préférence en polypropylène tricoté et présente avantageusement une structure ouverte, c'est-à-dire pourvue d'ouvertures délimitées par différents jeux de fils. Selon l'invention, l'élément allongé 2 est sensiblement souple, c'est-à-dire qu'il est suffisamment souple pour d'une part, éviter un phénomène de cisaillement des tissus biologiques après sa mise en place au sein du corps, et d'autre part, lui permettre de prendre une configuration repliée telle que celle illustrée sur la figure 1. L'élément allongé 2 est également suffisamment souple pour suivre les éventuels mouvements des tissus biologiques environnants après son implantation.

L'élément allongé 2 comprend, selon l'invention, une partie centrale 3 destinée à être positionnée sous l'urètre en guise de soutènement lorsque l'élément allongé 2 est en position fonctionnelle dans le corps, tel que cela est illustré sur la figure 1. L'élément allongé s'étend, suivant une direction longitudinale X-X', entre deux extrémités 4 et 5, situées de part et d'autre de la partie centrale 3. Selon une caractéristique essentielle de l'invention, les extrémités 4 et 5 de l'élément allongé 2 sont positionnées relativement à la partie centrale 3, de telle sorte que lorsque l'élément allongé 2 est en position fonctionnelle (figure 1), sa partie centrale 3 se trouvant alors sensiblement au droit de l'urètre dans une configuration classique de soutènement, l'une des extrémités 4 de l'élément allongé 2 est disposée entre le muscle obturateur interne droit OID et le muscle obturateur externe droit OED, l'autre extrémité 5 étant disposée entre le muscle obturateur interne gauche OIG et le muscle obturateur externe gauche OEG.

L'élément allongé 2 est ainsi avantageusement dimensionné de telle manière que lorsque sa partie centrale 3 est positionnée sous l'urètre, ses extrémités 4, 5 puissent être positionnées entre les muscles obturateurs interne et externe droit et gauche sans générer d'une part une mise en tension de l'élément allongé 2 et d'autre part un relâchement excessif de ce dernier, susceptible d'entraîner la formation de plis sur sa longueur.

La longueur de l'élément allongé 2 est ainsi avantageusement comprise entre 100 et 140 millimètres et de préférence de l'ordre de 130 millimètres,

De façon particulièrement avantageuse, l'implant prothétique 1 est simplement posé, sans tension, c'est-à-dire qu'il ne requiert aucun moyen d'ancrage positif pénétrant dans les tissus pour assurer sa mise en place. L'implant prothétique 1 constitue ainsi avantageusement une prothèse rétro-obturatrice posée.

En outre, l'élément allongé 2 n'effectue avantageusement aucune mise en tension de l'urètre, réalisant ainsi un simple soutènement de l'urètre et non une suspension de ce dernier. En effet, par ré-habitation cellulaire, un tissu cicatriciel vient se former autour de l'implant prothétique 1, précisément autour de l'élément allongé 2, formant ainsi une matrice de tissu cicatriciel renforcée par l'implant prothétique, ladite matrice étant susceptible de contenir et de s'opposer aux forces d'expulsion des urines. La structure ouverte de la bandelette joue donc un rôle crucial dans la mesure où elle favorise la formation du tissu cicatriciel et donc l'accrochage *« naturel* », c'est-à-dire sans moyens d'ancrage « *positifs* », de la bandelette sur toute sa longueur.

L'implant prothétique 1 est avantageusement adapté pour pouvoir être mis en place par voie vaginale exclusive, en réalisant une unique incision au niveau du vagin 6.

De façon à assurer un maintien efficace et confortable de l'urètre tout en minimisant le risque de rejet, l'élément allongé 2 présente avantageusement une largeur sensiblement comprise entre 10 et 20 millimètres, de préférence de l'ordre de 15 millimètres.

Dans un mode de réalisation particulièrement avantageux et qui constitue d'ailleurs une invention à part entière, l'élément allongé 2 est pourvu, à ses extrémités, de moyens de maintien 7 en position, non-pénétrants (figure 2). Les moyens de maintien 7 sont avantageusement destinés à assurer, par simple appui et friction sur les tissus biologiques, et sans pénétrer ces derniers, une immobilisation suffisante de l'élément allongé 2 pour permettre la ré-habitation cellulaire autour de l'implant prothétique 1. Ainsi, bien que la formation du tissu cicatriciel soit généralement rapide, il peut arriver qu'un jeu excessif de l'implant prothétique ait pour effet d'augmenter de façon notable le temps de cicatrisation. Les moyens de maintien 7 permettent ainsi de limiter le jeu de la bandelette tout en permettant à cette dernière de suivre, en raison de sa souplesse, les éventuels mouvements de faible amplitude des tissus environnants.

De façon particulièrement avantageuse, les moyens de maintien 7 sont formés par une structure suffisamment souple pour se replier lors de la mise en place de l'implant prothétique 1, et se déployer lorsque ledit implant est en position fonctionnelle. Ainsi, il est particulièrement intéressant, lors de la mise en place de l'implant et lors de son déplacement dans l'espace de dissection, d'éviter que l'implant ne s'oppose au mouvement, par exemple en venant s'accrocher sur des tissus biologiques. En revanche, dès lors que l'implant prothétique 1 est mis en place dans sa position fonctionnelle, le déploiement des moyens de maintien 7 permet de retenir l'élément allongé 2 en position, en favorisant le contact avec les tissus biologiques environnants.

Comme illustré sur les figures 2 et 3, les moyens de maintien 7 sont formés par des goussets 7A, 7B. Tel que cela est illustré sur la figure 3, les goussets 7A, 7B présentent, en position déployée, un rebord de retenue 8A, 8B de nature à constituer un obstacle supplémentaire à l'encontre de la mobilité de l'élément allongé 2. Les goussets 7A, 7B assurent ainsi individuellement le maintien en position des extrémités 4, 5 de l'élément allongé 2 par rapport aux tissus biologiques environnants.

Les goussets 7A, 7B sont montés en opposition aux extrémités 4, 5 de l'élément allongé 2, de manière à assurer, par action mutuelle, l'immobilisation de l'implant. Ainsi, les rebords de retenue 8A, 8B étant disposés en regard l'un de l'autre, tel que cela est illustré sur la figure 2, ils empêchent le déplacement de l'élément allongé 2 suivant la direction longitudinale X-X'.

Les goussets 7A, 7B s'ouvrent du côté de la partie centrale 3 de l'élément allongé 2 au niveau d'entrées 9A, 9B permettant le passage d'un outil de pose (non représenté) de l'implant prothétique (1), pourvu d'un organe de contact tel que la pointe de ciseaux courbes, destiné à venir en appui contre l'implant prothétique 1. Ainsi, les goussets 7A, 7B peuvent servir de moyens de réception de l'outil de pose, au sein desquels ledit outil de pose et plus précisément l'organe de contact, par exemple la pointe des ciseaux courbes, peut venir prendre appui pour pousser l'implant prothétique 1 à travers l'espace de dissection, facilitant ainsi sa mise en place.

L'élément allongé 2 et les goussets 7A, 7B sont réalisés à partir de la même bandelette textile. Ainsi, tel que cela est illustré sur la figure 4, l'élément allongé 2 est avantageusement pourvu, à ses extrémités 4, 5, de deux ailes 10A, 10B aptes à être rabattues au niveau d'une pliure 11 de manière à former les goussets 7A, 7B. De façon préférentielle, les ailes 10A, 10B sont rabattues de telle sorte que l'un des bord 10' des ailes 10A, 10B se superpose sur un bord correspondant 10" de la partie principale 2A de l'élément allongé 2 en vue d'être cousu avec ce dernier (figure 4). Ce mode de réalisation des goussets n'est bien évidemment donné qu'à titre illustratif et l'on pourrait également imaginer d'autres configurations sans sortir du cadre de l'invention.

Selon une caractéristique particulièrement avantageuse de l'invention, les goussets 7A, 7B sont profilés en forme de pointe, de préférence arrondie, de manière à faciliter la pénétration et le déplacement de l'implant prothétique 1 dans l'espace de dissection.

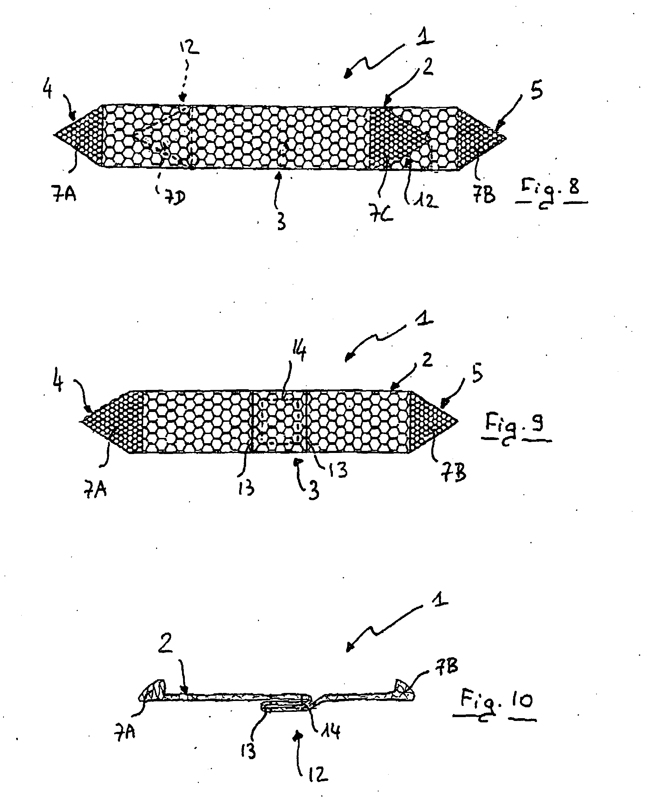

Selon un autre mode de réalisation de l'invention, illustré sur les figures 8 à 10, l'implant prothétique 1 comporte des moyens d'ajustement 12 de sa longueur, permettant d'ajuster la longueur de l'élément allongé 2 à l'anatomie de la patiente.

Selon une variante préférentielle de réalisation illustrée sur la figure 8, les moyens d'ajustement 12 comportent au moins un premier gousset d'ajustement 7C disposé entre les deux goussets 7A, 7B d'extrémité, vers l'un desdits goussets 7B d'extrémité, et apte à se substituer audit gousset 7B d'extrémité pour former un moyen de maintien de l'implant prothétique 1, de manière à raccourcir ce dernier.

En particulier, afin de raccourcir l'implant prothétique 1 de manière à l'adapter à l'anatomie de la patiente, le chirurgien peut sectionner, à l'aide de ciseaux, le gousset 7B d'extrémité situé à proximité du premier gousset d'ajustement 7C, l'implant prothétique 1 étant alors maintenu par le gousset 7A d'extrémité et le premier gousset d'ajustement 7C.

Il est également envisageable, selon une autre variante de réalisation non représentée aux figures, de réaliser un implant prothétique 1 dont les moyens d'ajustement 12 comportent deux goussets d'ajustement, à savoir un premier et un deuxième goussets d'ajustement 7C, 7D, disposés entre les goussets 7A, 7B d'extrémité de préférence symétriquement de part et d'autre de la partie centrale 3.

Il est bien évidemment envisageable d'augmenter encore le nombre des goussets d'ajustement de manière à permettre un ajustement encore plus fin de la longueur de l'élément allongé 2.

Selon une autre variante de réalisation illustrée sur la figure 9, les moyens d'ajustement 12 comportent au moins une pliure 13 ménagée sur la largeur de l'implant prothétique 1, de préférence dans la partie centrale 3, de manière à former une pliure transversale sensiblement perpendiculaire à la direction longitudinale X-X'.

La pliure transversale 13 est avantageusement maintenue par un moyen de fixation 14 amovible, de manière à permettre l'allongement de l'implant prothétique 1 une fois le moyen de fixation 14 retiré.

De façon préférentielle, et tel que cela est illustré sur les figures 9 et 10, le moyen de fixation 14 amovible est une couture. L'implant prothétique 1, et notamment l'élément allongé 2 présente donc initialement, après sa fabrication, une longueur prédéterminée susceptible d'être augmentée en retirant le moyen de fixation 14, notamment la couture, de manière à permettre le dépliement de l'implant prothétique 1.

Ainsi, si au cours de la pose, le chirurgien estime que la bandelette textile est trop courte compte-tenu de l'anatomie de la patiente, il peut augmenter la longueur de cette dernière en sectionnant le fil de couture puis en tirant sur les extrémités 4, 5 de l'implant prothétique 1 de manière à l'étirer et à éliminer la pliure transversale 13.

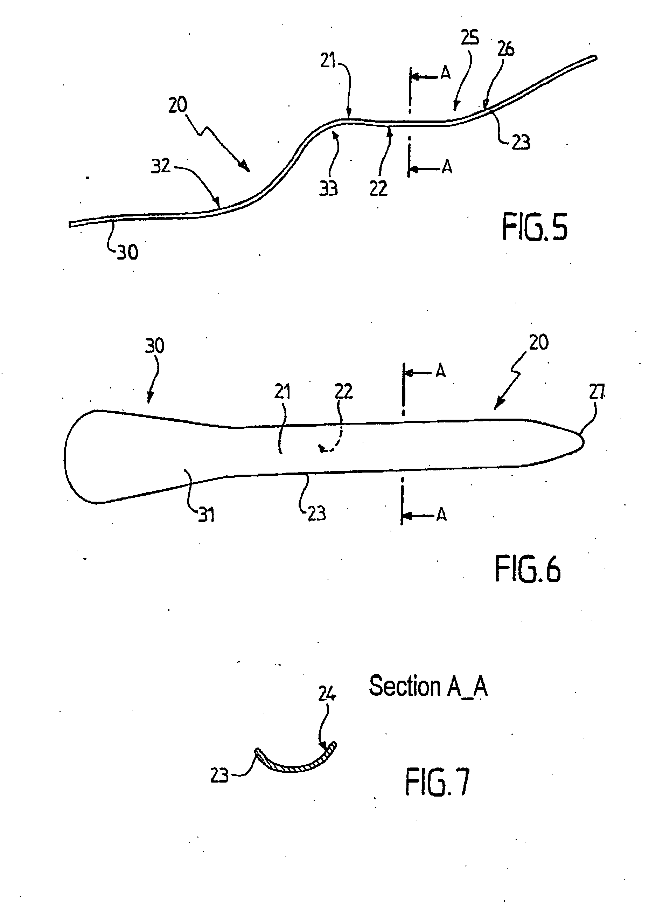

La figure 5 illustre un instrument chirurgical pour l'implantation d'un implant prothétique 1 de soutènement sous-urétral dans un espace de dissection. L'espace de dissection est avantageusement formé par un chemin d'accès ménagé de part et d'autre de l'urètre, orienté en haut, en dehors et en avant, sous la paroi vaginale, tout d'abord en direction de la banche ischio-pubienne puis en la contournant par en dessus, de manière à autoriser le passage de l'implant prothétique 1.

Au sens de l'invention, les directions des mouvements effectués sont données par rapport à un individu en position debout. Ainsi, *« en* haut » signifie vers la tête, « *en bas »* vers les pieds, « *en dedans »* vers l'intérieur du corps, « *en dehors* » vers l'extérieur du corps, « *en avant »* signifie dans le sens de la marche et « *en arrière* » dans le sens contraire de la marche.

L'instrument chirurgical 20 comporte un support de guidage 21 sensiblement allongé et destiné à être introduit dans l'espace de dissection susmentionné. Selon l'invention, le support de guidage 21 est spécifiquement adapté pour que l'implant prothétique 1 puisse glisser le long dudit support de guidage 21 vers sa position fonctionnelle dans le corps.

Le support de guidage 21 présente ainsi avantageusement une surface de glissement 22. L'implant prothétique 1 est alors destiné à être porté au contact de ladite surface de glissement 22 et à se déplacer le long de cette dernière, utilisée d'une part comme support et d'autre part comme guide.

Un tel support de guidage 21 permet ainsi au chirurgien de conduire des interventions facilement reproductibles, sans risque d'erreur.

De façon particulièrement avantageuse, le support de guidage 21 est formé, tel que cela est représenté sur les figures 5 et 6, par une lame 23. De façon encore plus préférentielle, la lame 23 présente une section transversale en « *U* » (figure 7) de manière à former une glissière 24 pour l'implant prothétique 1. Cette forme de la lame 23 permet ainsi d'améliorer encore d'avantage le guidage de l'implant prothétique 1 vers sa position fonctionnelle. Selon un autre aspect avantageux de l'invention, la lame 23 est conformée géométriquement de manière à s'adapter à l'anatomie de l'espace de dissection, c'est-à-dire qu'elle va d'une part pouvoir être facilement introduite au sein de l'espace de dissection et d'autre part sensiblement épouser les tissus délimitant cet espace.

A cet effet, la lame 23, présente avantageusement, sur sa longueur, au moins une première portion courbe 25.

L'espace de dissection étant délimité en bas et en dehors par la branche ischio-pubienne, la première portion courbe 25 présente avantageusement une courbure sensiblement complémentaire de la courbure osseuse de la branche ischio-pubienne. De cette façon, la partie concave 26 de ladite première portion courbe 25 vient sensiblement épouser la surface interne convexe de la branche ischio-pubienne. L'instrument chirurgical 20 constitue alors, avantageusement, un applicateur anatomique permettant d'introduire l'implant prothétique 1 selon un trajet très précis, situé à proximité directe de l'instrument chirurgical, et donc sans risque d'endommagement des tissus environnants.

Selon un aspect particulièrement intéressant, l'instrument chirurgical 20 comporte avantageusement une partie de préhension 30 formée par un prolongement 31 de la lame 23, afin de faciliter la manipulation de l'instrument par le chirurgien, la partie de préhension 30 étant de préférence sensiblement plus large que la lame 23. Il est ainsi préférable, dans le cas de techniques chirurgicales peu intrusives, de limiter les dimensions de la partie active de l'instrument, à savoir la lame 23, tout en facilitant le geste chirurgical. A cet effet, la partie de préhension 30 présente avantageusement une courbure longitudinale destinée à faciliter l'introduction du support de guidage 21, précisément de la lame 23, dans l'espace de dissection. La partie de préhension 30 forme alors une deuxième portion courbe 32 (figure 5). L'instrument chirurgical 20 présente ainsi une double courbures, lui conférant un caractère anatomique amélioré.

De façon préférentielle, et tel que cela est représenté sur la figure 5, la première et la deuxième portions courbes 25, 32 présentent des courbures orientées dans le même sens.

Avantageusement, la lame 23 comporte une troisième portion courbe 33 située entre la première et la deuxième portions courbes 25, 32 et qui présente une courbure inversée par rapport auxdites première et deuxième portions courbes. Cette troisième portion courbe 33 permet ainsi avantageusement de contourner les tissus biologiques lors de l'introduction de l'instrument chirurgical 20 dans l'espace de dissection.

Afin de favoriser la pénétration et le déplacement de l'instrument chirurgical 20 dans l'espace de dissection, tout en présentant un caractère atraumatique pour les tissus biologiques, le bord d'attaque 27 de la lame 23 est en forme de pointe émoussée (figure 6). De façon préférentielle, la lame 23 est en matériau sensiblement rigide, par exemple en polymère rigide ou en métal (acier inoxydable), mais on peut également envisager la possibilité que la lame 23 soit formée par un matériau déformable et/ou malléable en vue de conserver la possibilité d'adapter la forme anatomique de l'instrument à chaque situation particulière.

De façon préférentielle, la longueur de l'instrument chirurgical 20 est sensiblement comprise entre 120 et 180 millimètres et de préférence de l'ordre de 160 millimètres.

L'instrument chirurgical 20 comprend ainsi d'une part la partie de préhension 30, de longueur de préférence sensiblement égale à 50 millimètres et d'autre part le support de guidage 21, avantageusement formé par la lame 23, de longueur préférentiellement de l'ordre de 110 millimètres.

Par ailleurs, la largeur optimale du support de guidage 21 est préférentiellement de l'ordre de 15 millimètres, la partie de préhension 30 présentant de préférence une largeur supérieure, de l'ordre de 25 millimètres.

Un kit d'introduction de l'implant prothétique 1 comprend d'une part ledit implant prothétique 1, selon l'invention et d'autre part l'instrument chirurgical (20) tels que décrits précédemment.

Avantageusement, le kit d'introduction peut également comporter l'outil de pose de l'implant prothétique (1) dont les caractéristiques sont décrites ci-dessus.

Une méthode chirurgicale de traitement de l'incontinence urinaire chez la femme va maintenant être décrite en s'appuyant sur les figures 1 à 7.

Pour pratiquer ladite méthode, la patiente est installée en position gynécologique, les cuisses hyperfléchies.

La méthode chirurgicale comprend tout d'abord une étape (a) d'incision, au cours de laquelle on réalise, sous anesthésie locale, loco-régionale ou encore sous anesthésie générale, une incision sur la paroi vaginale antérieure, sous le méat urinaire de manière à permettre l'introduction de l'implant prothétique (1). Il s'agit préférentiellement d'une incision verticale médiane, d'environ 15 à 30 millimètres de longueur, réalisée 15 millimètres environ sous le méat urinaire.

La méthode chirurgicale comporte ensuite avantageusement une étape (b) de préparation au cours de laquelle le chirurgien ménage, à l'aide de ciseaux de dissection, un chemin d'accès de part et d'autre de l'urètre, sous la paroi vaginale, précisément à la face profonde de la paroi vaginale, et en direction de la branche ischio-pubienne, de manière à autoriser le passage de l'implant prothétique 1.

On réalise ainsi, à partir de l'incision et en direction de la branche ischio-pubienne, un premier trajet à droite de l'urètre et un deuxième trajet à gauche de l'ûrètre (et *vice-versa*), de telle sorte que le premier et le deuxième trajets forment le chemin d'accès.

De chaque côté de l'urètre, le geste réalisé est sensiblement le même, à savoir préférentiellement un mouvement en haut, en dehors et en avant, en direction de la branche ischio-pubienne afin de ménager les premier et deuxième trajets, de telle sorte que ces derniers soient sensiblement symétriques par rapport à l'urètre. Ce mouvement est ensuite poursuivi au-dessus de cette branche osseuse en gardant la même direction générale, de manière à contourner ladite branche osseuse.

Cette étape (b) de préparation permet ainsi d'éviter le risque de perforation d'organes nobles, en ménageant au préalable, avec précision, un chemin d'accès bien défini, susceptible d'être emprunté en toute sécurité pour assurer la mise en place de l'implant prothétique (1). L'implant prothétique (1) ne fait donc pas l'objet d'une mise en place en aveugle, contrairement à d'autres méthodes chirurgicales de l'art antérieur.

La méthode chirurgicale conforme à l'invention comporte ensuite une étape (c) de positionnement où l'on conduit chacune des extrémités 4, 5 de l'implant prothétique 1 le long du chemin d'accès correspondant ménagé au préalable, et dans laquelle on amène et on positionne chacune des extrémités 4, 5 entre le muscle obturateur interne et le muscle obturateur externe correspondant, traversant ou non la membrane obturatrice, mais sans ressortir à l'extérieur des trous obturateurs, c'est-à-dire, au sens de l'invention, sans traverser les trous obturateurs, ni le plan cutané.

On positionne ainsi une extrémité 4 de la bandelette formant l'implant prothétique 1 entre le muscle obturateur interne droit OID et le muscle obturateur externe droit OED, l'autre extrémité 5 étant disposée entre le muscle obturateur interne gauche OIG et le muscle obturateur externe gauche OEG. On fait ainsi passer la bandelette de part et d'autre de l'urètre (non représenté), au-dessus du muscle bulbo-caverneux MBC, au-dessus de la branche ischio-pubienne de l'os pubien OP, de manière à positionner les extrémités de la bandelette en arrière du muscle obturateur interne et au-dessus de l'aponévrose profonde.

Lors de cette étape (c) on vient également, naturellement, positionner la partie centrale (3) de l'élément allongé (2) sensiblement sous l'urètre, au droit de ce dernier, de telle sorte que ladite partie centrale (3) assure le soutènement de l'urètre.

De façon particulièrement avantageuse, lorsque la bandelette est positionnée correctement, on la laisse simplement reposer sur les tissus de l'espace de dissection sans la fixer. C'est donc la ré-habitation cellulaire qui va permettre la fixation progressive de la bandelette au sein d'un tissu cicatriciel.

Dans sa position fonctionnelle, l'implant prothétique 1 occupe ainsi avantageusement une forme en « V », avec un fort degré d'ouverture de telle sorte que l'implant s'étend presque horizontalement. Il n'existe donc pas de risque de perforation de la vessie V avec le type d'implant utilisé dans cette méthode.

De façon particulièrement avantageuse, la méthode comporte également une étape intermédiaire dans laquelle on introduit un instrument chirurgical 20 dans le chemin d'accès, ledit instrument chirurgical 20 comprenant un support de guidage 21 contre lequel on vient ensuite faire glisser l'implant prothétique 1 vers sa position fonctionnelle. L'instrument chirurgical 20 constitue ainsi avantageusement un applicateur anatomique, destiné à être glissé successivement dans chacun des deux trajets ménagés de chaque côté de l'urètre.

Lors de son insertion, la partie concave 26 de la première portion courbe 25 de l'instrument chirurgical 20 regarde en bas et en dehors et tourne autour de la branche ischio-pubienne en suivant sa convexité. Le chirurgien procède ensuite à la mise en place de l'implant prothétique 1, qui s'effectue, à partir de l'incision déjà réalisée dans la paroi vaginale, en poussant ce dernier vers sa position terminale ou fonctionnelle le long de la glissière 24 formée par la lame 23, c'est-à-dire sans qu'il soit nécessaire de tracter ledit implant prothétique 1 à partir d'une incision supplémentaire ménagée à un autre endroit du corps de la patiente.

A cet effet, le chirurgien utilise de préférence un objet (ou outil de pose) présentant une extrémité effilée, telle que la pointe de ciseaux courbes, pour saisir l'implant prothétique 1. En particulier, la pointe des ciseaux est enfilée dans l'un des goussets 7A, 7B disposés aux extrémités 4, 5 de l'implant prothétique 1, par exemple de la bandelette en polypropylène. La pointe des ciseaux est ensuite introduite dans l'un des deux trajets, où elle est guidée par le toboggan formé par l'applicateur, emmenant ainsi l'extrémité correspondante de la bandelette vers sa position terminale. Une fois la bandelette positionnée correctement, cette dernière est maintenue en position par la pointe des ciseaux pendant que l'applicateur est retiré. Les ciseaux ne sont retirés qu'ensuite, laissant ainsi la moitié de la bandelette dans le trajet. Une gestuelle sensiblement identique est reproduite de l'autre côté de l'urètre, afin de positionner la deuxième moitié de la bandelette.

A la suite de cette procédure, l'implant prothétique 1 est simplement posé, et n'est ni suspendu, ni fixé aux tissus biologiques par un quelconque moyen de fixation.

Ce sont avantageusement les moyens de maintien 7 qui assurent, suite à l'implantation de la prothèse, une immobilisation suffisante de cette dernière pour que la ré-habitation cellulaire puisse s'effectuer rapidement.

Une telle méthode présente l'avantage de permettre une intervention par voie vaginale exclusive, sans qu'il soit nécessaire de réaliser des incisions supplémentaires dans l'abdomen, dans l'aine, le pli crural ou à la face interne de la fesse en vue de tracter la bandelette. Cette méthode utilise ainsi la voie rétro-obturatrice.

En outre, la bandelette étant particulièrement courte, elle permet de limiter les risques de perforation d'organes nobles et donc d'éviter des contrôles supplémentaires, tels qu'une cystoscopie. Contrairement aux techniques antérieures, il n'est pas nécessaire de fixer la bandelette, et cette dernière permet également de limiter le risque de rejet en raison de sa faible longueur. La méthode de pose de la bandelette est en outre particulièrement facile et rapide à mettre en oeuvre et est également intéressante du point de vue de son innocuité. Cette méthode ne requiert ainsi aucune perforation de plans pariétaux, ce qui, ajouté à sa rapidité de réalisation, permet de limiter de façon notable les risques de perforation d'organes nobles ou de douleurs séquellaires.

### POSSIBILITE D'APPLICATION INDUSTRIELLE

L'invention trouve son application industrielle dans la conception et la fabrication d'implants prothétiques de traitement de l'incontinence urinaire.

## Revendications

1. Implant prothétique (1) de soutènement sous-urétral comprenant un élément allongé (2) sensiblement souple formé par une bandelette textile et pourvu :
- d'une partie centrale (3) destinée à être positionnée sous l'urètre en guise de soutènement lorsque l'élément allongé (2) est en position fonctionnelle dans le corps,
- de deux extrémités (4, 5) situées de part et d'autre de la partie centrale (3),
- et de moyens de maintien (7) en position de l'implant (1), disposés aux extrémités (4, 5), et réalisés à partir de ladite bandelette textile ledit implant (1) étant **caractérisé en ce que** lesdits moyens de maintien (7) forment des goussets (7A, 7B) positionnés du même côté de l'élément allongé (2) et sont montés en opposition aux extrémités (4, 5), l'entrée (9A, 9B) desdits goussets (7A, 7B) se situant du côté de la partie centrale (3) de l'élément allongé (2).

2. Implant (1) selon la revendication 1 **caractérisé en ce que** les moyens de maintien (7) sont destinés à assurer, par simple appui et friction sur les tissus biologiques, une immobilisation suffisante de l'élément allongé (2) pour permettre la ré-habitation cellulaire autour de l'implant (1).

3. Implant (1) selon la revendication 1 ou 2 **caractérisé en ce que** les moyens de maintien (7) sont non-pénétrants.

4. Implant prothétique selon l'une des revendications 1 à 3 **caractérisé en ce que** l'élément allongé (2) est pourvu de deux ailes (10A, 10B), disposées à ses extrémités (4, 5), et aptes à être rabattues de manière à former les goussets (7A, 7B).

5. Implant prothétique selon l'une des revendications 1 à 4 **caractérisé en ce que** les goussets (7A, 7B) sont profilés en forme de pointe de manière à faciliter la pénétration et le déplacement de l'implant (1) dans un espace de dissection.

6. implant prothétique selon l'une des revendications précédentes **caractérisé en ce que** la longueur de l'élément allongé (2) est sensiblement comprise entre 100 et 140 mm, de préférence de l'ordre de 130 mm.

7. Implant prothétique selon l'une des revendications précédentes **caractérisé en ce que** la largeur de l'élément allongé (2) est sensiblement comprise entre 10 et 20 mm, de préférence de l'ordre de 15 mm.

8. Implant prothétique selon l'une des revendications précédentes, **caractérisé en ce qu'**il comporte des moyens d'ajustement (12) de sa longueur.

## Claims

1. Prosthetic implant (1) for sub-urethral support, comprising a substantially supple elongate element (2) formed of a textile tape and provided:
- with a central part (3) intended to be positioned under the urethra by way of a support when the elongate element (2) is in the functional position within the body,
- with two ends (4, 5) situated one on each side of the central part (3),
- and with means (7) for holding the implant (1) in position, these holding means being positioned at the ends (4, 5) and made from the said textile tape,
the said implant (1) being **characterized in that** the said holding means (7) form pockets (7A, 7B) positioned on the same side of the elongate element (2) and are in opposition at the ends (4, 5), the entrance (9A, 9B) to the said pockets (7A, 7B) being situated on the side of the central part (3) of the elongate element (2).

2. Implant (1) according to Claim 1, **characterized in that** the holding means (7) are intended, simply by pressing and by friction against the biological tissues, to immobilize the elongate element (2) sufficiently to allow cell re-growth around the implant (1).

3. Implant (1) according to Claim 1 or 2, **characterized in that** the holding means (7) are nonpenetrating.

4. Prosthetic implant according to one of Claims 1 to 3, **characterized in that** the elongate element (2) is provided with two wings (10A, 10B) positioned at its ends (4, 5) and that can be folded back to form the pockets (7A, 7B).

5. Prosthetic implant according to one of Claims 1 to 4, **characterized in that** the pockets (7A, 7B) are shaped into the form of a point to make the implant (1) easier to insert and move around in a dissection space.

6. Prosthetic implant according to one of the preceding claims, **characterized in that** the length of the elongate element (2) is comprised essentially between 100 and 140 mm and is preferably of the order of 130 mm.

7. Prosthetic implant according to one of the preceding claims, **characterized in that** the width of the elongate element (2) is comprised substantially between 10 and 20 mm, and is preferably of the order of 15 mm.

8. Prosthetic implant according to one of the preceding claims, **characterized in that** it comprises means (12) of adjusting its length.

## Patentansprüche

1. Prothetisches Implantat (1) zur suburethralen Unterstützung, das ein im Wesentlichen biegsames, längliches Element (2) enthält, welches von einem Textilband geformt wird und versehen ist mit:
- einem Mittelbereich (3), der dazu bestimmt ist, unter der Harnröhre als Unterstützung positioniert zu werden, wenn das längliche Element (2) in der funktionellen Stellung im Körper ist,
- zwei Enden (4, 5), die sich zu beiden Seiten des Mittelbereichs (3) befinden,
- und Halterungsmittel (7) zur Beibehaltung der Stellung des Implantats (1), die an den Enden (4, 5) angeordnet und ausgehend vom Textilband hergestellt sind,
wobei das Implantat (1) **dadurch gekennzeichnet ist, dass** die Halterungsmittel (7) Zwickel (7A, 7B) bilden, die auf der gleichen Seite des länglichen Elements (2) positioniert und einander entgegengesetzt an den Enden (4, 5) montiert sind, wobei der Eingang (9A, 9B) der Zwickel (7A, 7B) sich auf der Seite des Mittelbereichs (3) des länglichen Elements (2) befindet.

2. Implantat (1) nach Anspruch 1, **dadurch gekennzeichnet, dass** die Halterungsmittel (7) dazu bestimmt sind, durch einfache Auflage und Reibung auf den biologischen Geweben eine ausreichende Immobilisierung des länglichen Elements (2) zu gewährleisten, um die Wiederansiedlung von Zellen um das Implantat (1) herum zu erlauben.

3. Implantat (1) nach Anspruch 1 oder 2, **dadurch gekennzeichnet, dass** die Halterungsmittel (7) nicht eindringend sind.

4. Prothetisches Implantat nach einem der Ansprüche 1 bis 3, **dadurch gekennzeichnet, dass** das längliche Element (2) mit zwei Flügeln (10A, 10B) versehen ist, die an seinen Enden (4, 5) angeordnet sind und umgeklappt werden können, um die Zwickel (7A, 7B) zu bilden.

5. Prothetisches Implantat nach einem der Ansprüche 1 bis 4, **dadurch gekennzeichnet, dass** die Zwickel (7A, 7B) ein Profil in Form einer Spitze haben, um das Eindringen und die Verschiebung des Implantats (1) in einem Dissektionsbereich zu erleichtern.

6. Prothetisches Implantat nach einem der vorhergehenden Ansprüche, **dadurch gekennzeichnet, dass** die Länge des länglichen Elements (2) im Wesentlichen zwischen 100 und 140 mm, vorzugsweise in der Größenordnung von 130 mm liegt.

7. Prothetisches Implantat nach einem der vorhergehenden Ansprüche, **dadurch gekennzeichnet, dass** die Breite des länglichen Elements (2) im Wesentlichen zwischen 10 und 20 mm und vorzugsweise in der Größenordnung von 15 mm liegt.

8. Prothetisches Implantat nach einem der vorhergehenden Ansprüche, **dadurch gekennzeichnet, dass** es Anpassungsmittel (12) zur Anpassung seiner Länge enthält.
